# EUROPEAN PATENT SPECIFICATION

(11) **EP 2 424 476 B1**
(45) Date of publication and mention of the grant of the patent: **07.05.2014**
(21) Application number: 10769135.4
(22) Date of filing: 23.04.2010
(51) Int. Cl.: A61F 9/08, A61F 2/14, A61N 1/00

(54) **RETINAL PROSTHESIS**
RETINAPROTHESE
PROTHÈSE RÉTINIENNE

(30) Priority: 27.04.2009 AU 2009901812
(43) Date of publication of application: 07.03.2012
(73) Proprietor: National ICT Australia Limited, Eveleigh, NSW 2015 (AU); The Bionics Institute of Australia, East Melbourne, Victoria 3002 (AU); Centre for Eye Research Australia Limited, East Melbourne VIC 3002 (AU)
(72) Inventor: NG, David, CheeKeong, Pascoe Vale South VIC 3044 (AU); BAI, Shun, Parkville VIC 3052 (AU); SKAFIDAS, Efstratios, Thornbury VIC 3071 (AU); ALLEN, Penelope, Kew VIC 3101 (AU); WILLIAMS, Chris, East Melbourne VIC 3002 (AU); MEFFIN, Hamish, Braybrook VIC 3019 (AU); MCCOMBE, Mark, Kew VIC 3101 (AU); HALPERN, Mark, East Malvern VIC 3145 (AU); BOYD, Clive, Stewart, Mooroolbark VIC 3138 (AU)
(74) Representative: Legg, Cyrus James Grahame
(86) International application number: PCT/AU2010/000474
(87) International publication number: WO 2010/124321

(56) References cited:
- US-A- 5 935 155
- US-A1- 2002 198 573
- US-A1- 2005 222 624
- US-A1- 2006 253 172
- US-A1- 2008 154 336
- NG, D. C. ET AL.: 'Wireless technologies for closed-loop retinal prostheses' JOURNAL OF NEURAL ENGINEERING vol. 6, 23 October 2009, pages 1 - 10

## Description

### Technical Field

The present invention relates to retinal prostheses, and in particular it relates to the transfer of electrical power and data from outside of the human body to such a prosthesis.

### Background of the Invention

A retinal prosthesis which uses electrical signals to stimulate the retina requires electrical power to function. The power demands of retinal prostheses are likely to increase as higher density electrode arrays are developed in future. A retinal prosthesis cannot practically be powered by wires from an external power supply, as any wired connection through the skin creates a risk of infection and causes discomfort to the user. Previous proposals for powering retinal prostheses include the use of optical and acoustic energy, however these solutions are ineffective due to high losses when these energy types pass through tissue.

Inductive coupling links have also been proposed for visual prostheses. Such proposals provide for an external primary coil which is placed in front of the eye, and a secondary internal coil inside the eye. However, the large separation between these coils and the small diameter of the internal coil result in low power transfer efficiency.

Surgical implantation of a prosthesis into the eye also presents particular difficulties due to the need to ensure ongoing integrity of the ocular structure, at least after surgical healing is complete. For example the sclera provides resistance to forces on the eye, helps to maintain intraocular pressure, and helps resist infection within the eye. Any implanted device must also be structured and positioned in a way to allow for the normal range of motion of the eyeball and the almost continual nature of eyeball movement.

US 2005/222624 discloses a retinal prosthesis with an inductive coil mounted to the side of the eye by means of a strap around the eye. Such outside-sclera arrangement requires full-sclerotomy which limits the interconnect width of the sclera piercing cable and may ultimately lead to complications such as infection.

### Summary of the Invention

According to a first aspect the present invention provides a retinal prosthesis, comprising: a retinal electrode array (150) configured to be implanted in the eye to stimulate the retina (400); a receiving coil (110) ; an electrical connection (170) between the receiving coil and the retinal electrode; and wherein the receiving coil is flexible and configured to conform to scleral curvature, when it is implanted; characterised in that the receiving coil (110) is configured to be implanted sub-sclerally to inductively receive power or data signals, or both.

The sub-scleral positioning of the receiving coil enables a retinal prosthesis to be implemented which has no wiring or tethers from the intraocular devices to the extraocular space. This allows for complete healing of the sclera after the device is implanted, which allows the sclera to perform its role providing a barrier to intraocular infection.

The retinal electrode array may be sub-retinal, for instance it may be located epi-retinally near the fovea in the macular region of the retina.

One or more retinal self-locking tacks may be used to mechanically secure the electrode array in the epi-retinal position. A retinal tack may also complete an electrical connection between the secondary coil and the electrode array. The retinal tack may be formed of an insulating material, such as a ceramic, and house a conductor that spans electrical contacts at each end of the tack.

In one example the retinal tack may be arranged to conduct power or data signals, or both, from the secondary coil through the choroid or retina, or both, to the electrode array. The insulating material of the retinal tack may minimise electrical current leakage between the secondary coil and the electrode array. The conductor of the retinal tack may carry data signals in both directions, to and from the retinal array.

Other locations for the retinal electrode array are also possible, for instance in the suprachoroidal space.

The receiving coil (generally nominated the 'secondary' coil below) may be intrinsically flexible. Alternatively, it may be formed or mounted on a flexible substrate.

The receiving coil may be located between the sclera and the choroid. It may be located supra-choroidally and epi-sclerally.

The present invention thus provides for an implanted secondary coil which does not require a full sclerotomy, and as a result does not result in an interconnect piercing the sclera.

The receiving coil may be connected to the electrode array by the use of flexible wiring. The flexible wiring may be implanted sub-sclerally (in the suprachoroidal layer) between the coil and the array (or a retinal tack). Alternatively, the wiring from the receiving coil may intrude through the choroid and the vitreous humor to the array. In this case it is preferred that the wiring does not pass through any part of the retina.

The receiving coil may receive power and data signals by inductive coupling with a transmitting coil.

The remote transmitting (primary) coil may be located externally to the body, for instance mounted on the surface of the skin.

In some situations it may be necessary or desirable to use one or more additional, intermediate coils to relay power or data signals, or both, from the remote transmitting (primary) coil to the receiving (secondary) coil. For instance a first intermediate coil may be implanted in the zygomatic bone for receiving power from an external primary coil. A second intermediate coil may be implanted in the orbit of the ocular region for inductively relaying power from the first intermediate coil to the secondary. The first and second intermediate coils may be in wired connection, which improves transmission efficiency.

### Brief Description of the Drawings

Examples of the invention will now be described with reference to the accompanying drawings, in which:
Figs. 1(a) and 1(b) are diagrams that illustrate the anatomical positioning of a secondary coil for effecting an inductive power link to a retinal implant.
Fig. 2(a) is a diagram that illustrates the location of an external primary coil.
Fig. 2(b) is a diagram that illustrates the relative locations of the external primary coil of Fig. 2(a), two internal intermediate coils, and the internal secondary coil of Figs. 1.
Fig. 3(a) is cross-section of an eye from the side, showing the location of a retinal electrode array and the relative locations of the external primary coil and the internal secondary coil that are in front of the plane of the section, and the route of the flexible wiring interconnecting the internal secondary coil and the retinal electrode array.
Fig. 3(b) is a magnified section through the layers of the eyeball showing the locations of the internal secondary coil and the flexible wiring connected to it.
Fig. 3(c) is a magnified section through the layers of the eyeball of Fig. 3(b) showing the locations of the retinal electrode array, a retinal tack and the flexible wiring from the internal secondary coil.
Fig. 4(a) is cross-section of an eye from the side, showing the location of a retinal electrode array and the relative locations of the external primary coil and the internal secondary coil that are in front of the plane of the section, and an alternative route of the wiring interconnecting the internal secondary coil and the retinal electrode array.
Fig. 4(b) is a magnified section through the layers of the eyeball of Fig. 4(a) showing the locations of the internal secondary coil and the wiring connected to it.
Figs. 5(a) and (b) show a variation of the arrangement of Figs. 4 where the internal secondary coil is mounted on a flexible scleral buckle that is positioned epi-sclerally.
Fig. 6 is a diagram of the eyeball showing the location of the surgical incision required for a trans-vitreous connection to be made between the internal secondary coil and the retinal electrode array.
Fig. 7 is a variation on Fig. 2(b) where there is only a single intermediate coil.
Fig. 8(a) is a diagram of a first configuration for a power transfer coil.
Fig. 8(b) is a diagram of a second configuration for a power transfer coil.

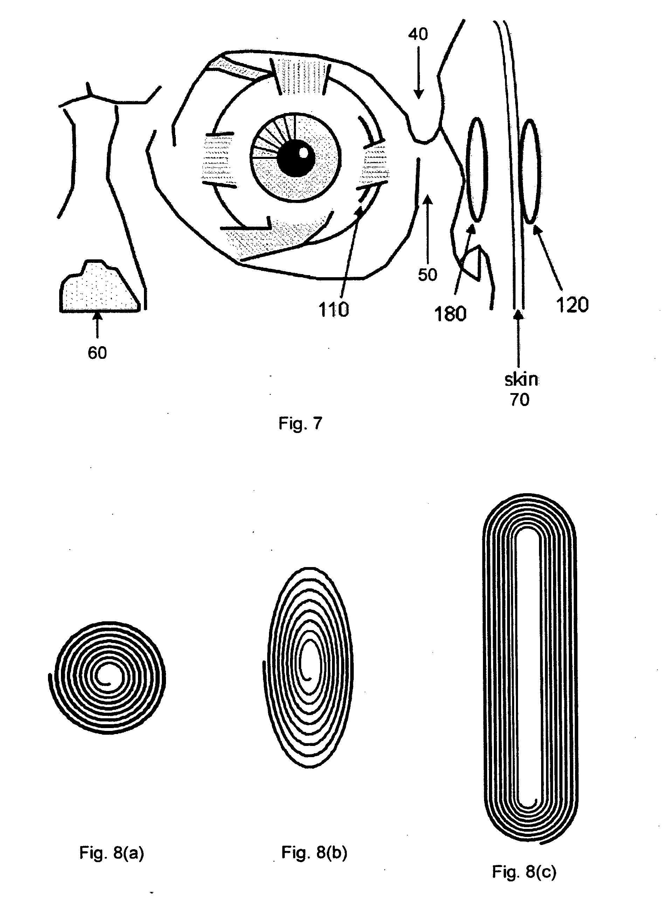

Fig. 8(a) is a diagram of a third configuration for a power transfer coil.

### Best Modes of the Invention

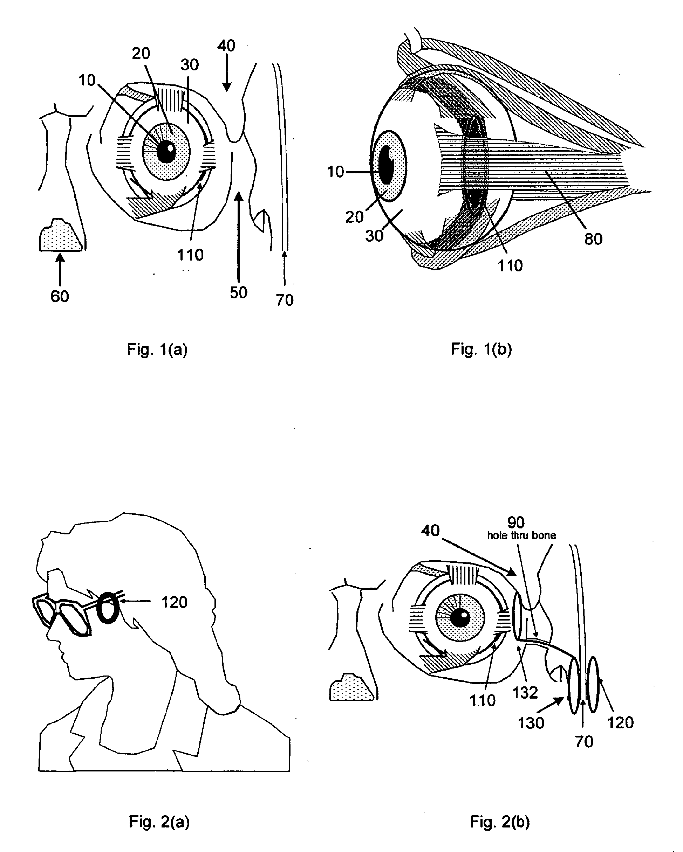

Referring first to Figs. 1(a) and 1(b), these diagrams illustrate the anatomical location of a secondary coil 110 for effecting an inductive power link to a retinal implant (not shown). Fig. 1(a) shows the eye ball from the front, and Fig. 1(b) from the side, with the pupil 10, iris, 20 and the sclera (white of the eye) 30. The bony structure around the eye is also indicated at 40 in Fig. 1(a), as is the zygomatic bone 50, the nose 60 and the skin on the temple 70. Several muscles that move the eye (such as the ciliary muscle 80) are also shown in both drawings but they are not important for understanding the location of the secondary coil 110. The secondary coil is shown at the side of the eye but is implanted beneath the sclera 30, and would not be visible to an observer.

Fig. 2(a) illustrates the location of an external primary coil 120, close to the temple. The primary coil 120 is located externally on or near the skin 70, and is connected to a power source (not shown). The secondary coil 110 is connected to a retinal implant (not shown) inside the eye and is located on a flexible substrate, that is implanted at a location under the sclera.

An inductive link operates by a time-varying electrical current flowing in a primary coil inducing a current in a secondary coil, provided it is located in sufficiently close proximity to the primary coil. In this case, the primary coil 120 is located outside the eye, while the secondary coil 110 is placed inside the eye. The primary coil 120 is connected to a power source via an electrical connection and electronics that drive the current in the coil. The secondary coil 110 is connected to an implanted device via electronics which recover the energy from the inductive link to supply power to the implant. The inductive link removes the need for a physical (wired) electrical connection between an external power source and the implanted device.

An inductive power link for a retinal prosthesis, must take into account the fact that the secondary coil 110 is constrained by the physical and biological environment of the eye, and the high power consumption of the implanted device. The aim is to deliver the required power without exceeding safety limits.

The secondary coil 110 is formed or mounted upon a flexible substrate which is surgically implanted in the supra-choroidal space. In particular the secondary coil 110 is provided on a thin flexible circuit, or substrate, much like a scleral buckle. This entire device is surgically implanted under the sclera, between the sclera and the choroid in the suprachoroidal space. Furthermore, the secondary coil is located near the ciliary muscle 80 (see Fig. 1(b)), away from the retina, to prevent injury to the delicate retina 400 layer.

Fig. 2(b) illustrates the relative locations of the external primary coil 120, two internal intermediate coils 130 and 132, as well as the internal secondary coil 110. The intermediate coils 130 and 132 are used to improve the power transfer efficiency between the primary coil 120 and the secondary coil 110. The first intermediate coil 130 sits on the zygomatic bone 50 of the skull immediately adjacent the external primary 120. The second intermediate coil 132 sits in the orbit of the ocular region immediately adjacent the secondary 110. These two coils are in circuit with each other via a hole 90 through the zygomtic bone.

Due to the relatively small distance between the secondary coil 110 and the second intermediate coil 132, and the relatively small distance between the primary coil 120 and the first intermediate coil 130, more efficient power transfer can be achieved. Moreover, such efficiency is achieved in a manner which provides wireless power transfer through the sclera 30, allowing the sclera to be maintained intact once healed from implantation surgery. Further, the relative positioning of second intermediate coil 132 and secondary 110 allows for normal movement of the eyeball, while maintaining high power transfer efficiency which is relatively unsusceptible to movements of the eye.

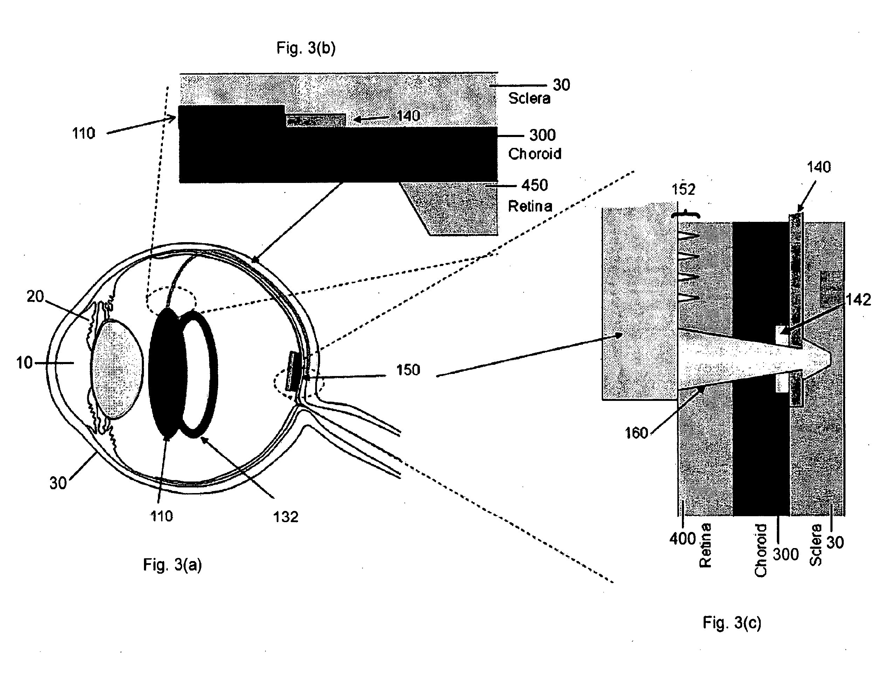

The retinal electrode array 150 is implanted epi-retinally near the fovea in the macular region of the retina. The retinal array is held in place by a self-locking retinal tack 160, seen in Fig. 3(c). Also shown in Fig. 3(c) are a number of retina stimulating electrodes 152 extending from the back of the electrode array 150 into the tissue of the retina.

Power is transferred from the secondary coil 110 to the retinal electrode array 150 by a physical connection comprising flexible circuit wiring 140. In Figs. 3(a) and (b) the flexible circuit wiring 140 is disposed along the suprachoroidal space between the secondary coil 110 and the retina, where is passes behind the retinal electrode array 150. To connect the power from the wiring 140 in the suprachoroidal space through the choroid 300 and retina 400 to the implant 150, it passes through a conductive path in the retinal tack 160.

The use of a retinal tack to both mechanically anchor the implant 150, and also provide an electrical connection from the wiring 140 to the implant, minimises the number of components piercing the retina 400and choroid 300.

It is envisaged that more than one tack may be required to adequately anchor implant 150. In the case where more than one tack 160 is used, some or all of the tacks may provide electrical connections to the wiring 140.

In order to prevent current leakage from the conductive path of the retinal tack 160, it is sheathed in non-conductive ceramic from the contact point 142 between the tack 160 and the flexible wiring 140. The conductor (not shown) of the retinal tack 160 then efficiently conveys power, and if required a data signal, from the electrical wiring 140 to the retinal implant 150 with minimal leakage. The power and data received by the electrode array 150 enables electrical stimuli to be applied via electrodes 152 to the nerve cells in the retina.

In order to locate the inductive coils 110, 120, 130 and 132 in and near the eye, the following surgical procedure is followed:
First, the flexible wiring 140 is inserted into the suprachoroidal space near the macular region of the eye.
Next, the secondary coil 110 is placed under the sclera 30.
Once the sclera 30 has recovered reasonably well, the retinal implant unit 150 is inserted by performing an incision at the pars plana region and securing it in place with the self-locking tacks 160.
Next, the intermediate coils 130 and 132 are anchored to the zygomatic bone with one coil in the ocular region and the other coil under the skin.

The intermediate coils 130 and 132 are connected via a physical wire which passes through a hole bored through the zygomatic bone, as shown in Fig. 2(b).

A wireless power link is then established from the intermediate coil 130 to the primary coil 120, outside the body. The primary coil could be carried by, or encased within, the frame of a pair of spectacles, as shown in Fig. 2(a).

The sub-scleral positioning of the secondary coil 110 enables a retinal prosthesis to be implemented which has no wiring or tethers from the intraocular device to the extraocular space. This arrangement allows the sclera 30 to completely heal after surgery, and return to its normal roles such as providing a barrier to intraocular infection and the like.

In some applications the interface between the retinal tack 160 and the flexible wiring 140 may suffer from long term reliability problems. These might be addressed by the implantation of a pre-connected and fully sealed connection between the two; rather than the use of retinal tacks.

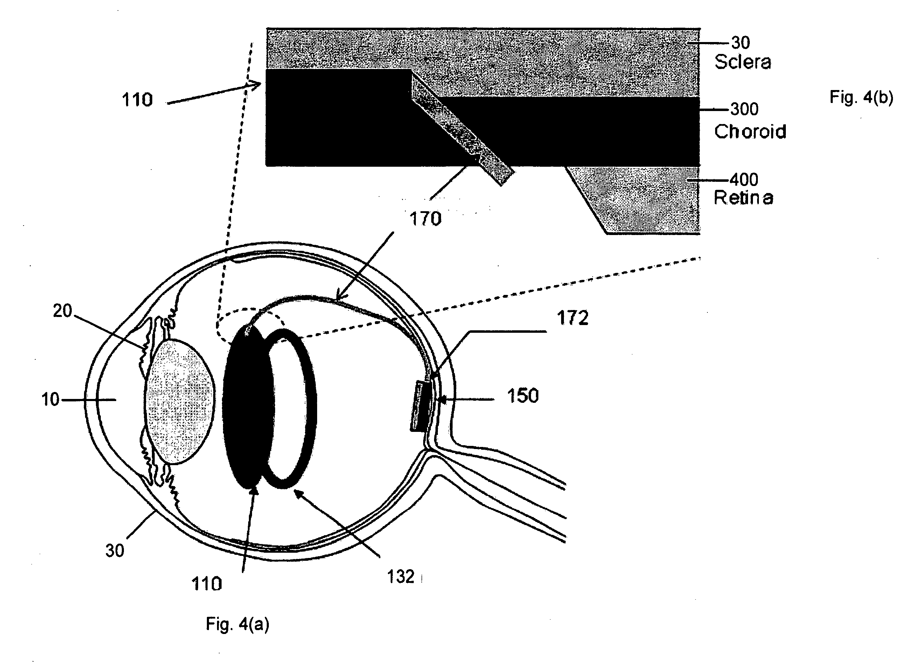

This might be achieved by use of a trans-vitreous link as shown in Figs. 4(a) and 4(b). In this variation flexible wiring 170 extends from the secondary coil 110 through the vitreous humour directly to a connection 172 on the exposed surface of the implanted device 150. This allows the electrical connection 172 between the flexible link 170 and the implant 150 to be fabricated in an integrated manner before implantation, and therefore will be highly reliable.

Another alternative involves placement of an intermediate coil onto the outer surface of the sclera 30. This effectively eliminates any relative motions between the secondary coil 110 and the intermediate coil 132 due to eye movements. This increases the efficiency of the inductive link since wireless power transfer is dependent on the amount of overlap between the coils.

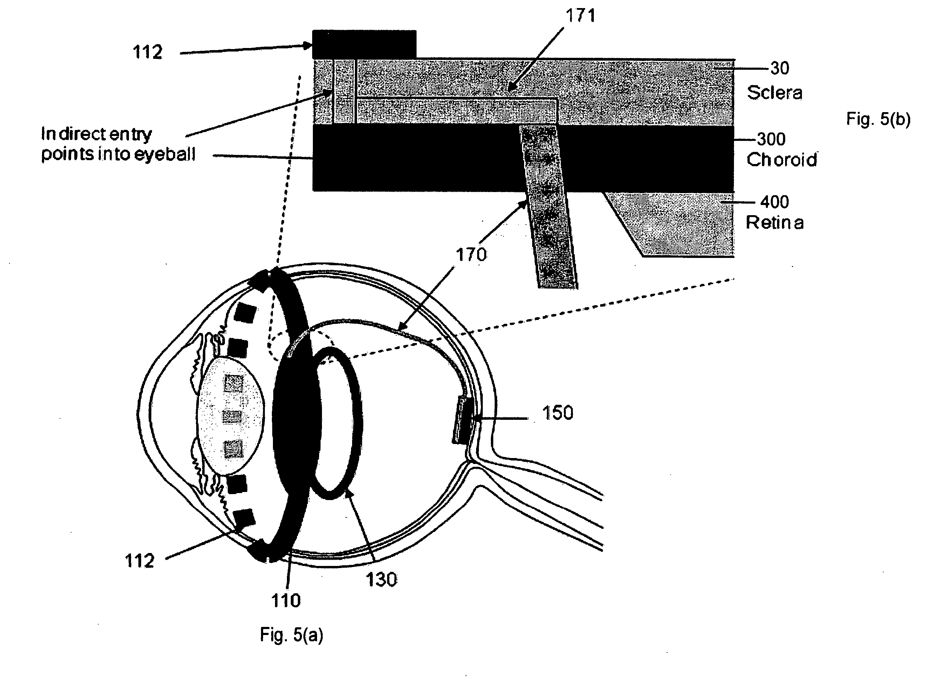

In the event that it is necessary for a transcleral link to be made, it is beneficial to run the link through the sclera 30, along between the sclera and the choroid 300 and then through the choroid, rather than penetrating directly through both layers of the eyeball. The link could run under the sclera 30 for up to half the perimeter of the eyeball before penetrating the choroid 300. This separation of the puncture through the sclera 30 and choroid 300 reduces the possibility of inner ocular infection and assists post surgical recovery. For instance the secondary coil 110 could be mounted on a sclera buckle 112 and the assembly be placed epi-sclerally, as shown in Fig. 5. The benefit of placing the secondary coil 110 epi-sclerally is to enable a larger diameter coil to be realised, thus improving power transfer efficiency between the primary and secondary coils. Depending on the size of the secondary coil, this modification can potentially replace the need for an intermediate coil.

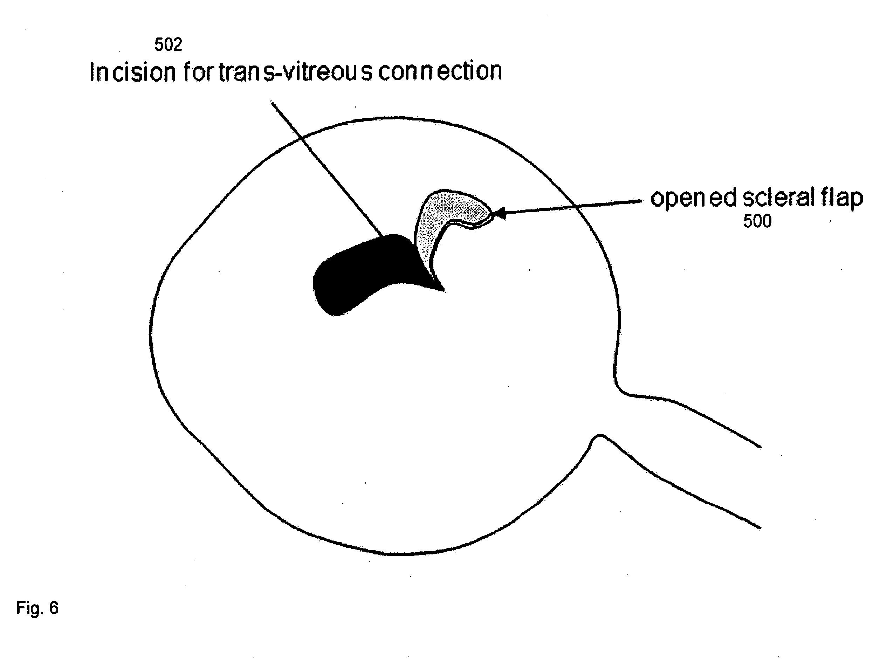

In order to implant the devices described with reference to Figs. 4 and 5, the following surgical procedure maybe performed:
A sclera flap 500 is cut above the lateral eye muscle and the flab folded backward to expose the choroid 300 below.
An incision 502 on the choroid 300 is then made, as shown in Fig. 6. The location of this incision should be made close to the pars plana region away from the retina 400 layer to avoid damage to the retina.
The implant 150 which has been integrated into the flexible trans-vitreous link 170 beforehand is then inserted through the incision.
Once the insertion is complete, the sclera flap 500 is closed.

In the case of Fig. 4(a), closure of flap 500 is achieved by it being sutured back to the surrounding sclera 30 and fully sealed; as shown in Fig. 4(b). For the arrangement of Fig. 5(a), it is necessary to leave part of the flap open to allow for a trans-scleral link 171 to be connected to the secondary coil 110 which is mounted on the scleral buckle 112.

Another alternative arrangement involves the use of a single intermediate coil 180 placed between the skin and the zygomatic bone, in the vicinity of a primary 120 and a secondary 110; see Fig. 7.

Many different configurations are possible for the power transfer coils 110, 120, 130, 132, 180. Generally, a coil consists of a continuous conductive track which spirals outwards resulting in a number of turns. The shape of the coil may be circular, oval or oblong as depicted in Figs. 8(a), (b) and (c) respectively. The amount of required power transfer and ease of surgical procedure are factors that are taken into account when determining the size and shape of the coils.

It will be appreciated that a sympathetic combination of coil configuration and location, surgical and patient friendly design, and reliable mechanical and electrical connections are required to implement feasible inductive power transfer to a retinal implant.

It will also be appreciated by those skilled in the art that numerous variations and modifications may be made to the invention as described above without departing from the scope of the invention as broadly defined by the claims. The present embodiments are, therefore, to be considered in all respects as illustrative and not restrictive.

## Claims

1. A retinal prosthesis, comprising: a retinal electrode array (150) configured to be implanted in the eye to stimulate the retina (400); a receiving coil (110); an electrical connection (170) between the receiving coil and the retinal electrode; and wherein the receiving coil is flexible and configured to conform to scleral curvature, when it is implanted;
**characterised in that** the receiving coil (110) is configured to be implanted sub-sclerally to inductively receive power or data signals, or both.

2. A retinal prosthesis according to claim 1, wherein the retinal electrode array (150) is configured to be located epi-retinally near the fovea in the macular region of the retina (400).

3. A retinal prosthesis according to claim 1 or 2, further comprising one or more retinal self-locking tacks (160) that are configured to facilitate mechanical securing of the electrode array (150) in the epi-retinal position, and also are configured to complete an electrical connection between the secondary coil (110) and the electrode array.

4. A retinal prosthesis according to claim 3, wherein each one of said retinal tacks (160) is formed of an insulating material, such as a ceramic, and houses a conductor that spans electrical contacts at each end of the tack.

5. A retinal prosthesis according to claim 3 or 4, wherein each one of said retinal tacks (160) is arranged to conduct power or data signals, or both, from the secondary coil (110) through the choroid (300) or retina (400), or both, to the electrode array (150).

6. A retinal prosthesis according to claim 4 or 5, wherein the conductor of each one of said retinal tacks (160) is configured to carry data signals in both directions, to and from the retinal array.

7. A retinal prosthesis according to claim 1, wherein the retinal electrode array (150) is configured to be located in the suprachoroidal space.

8. A retinal prosthesis according to any preceding claim, wherein the receiving coil (110) is intrinsically flexible.

9. A retinal prosthesis according to any of claims 1 to 7, wherein the receiving coil (110) is formed or mounted on a flexible substrate.

10. A retinal prosthesis according to claim 1, wherein the receiving coil (110) is configured to be located between the sclera (30) and the choroid (300), for example supra-choroidally and epi-sclerally, the receiving coil (110) being connected to the electrode array (150) by the use of flexible wiring, which is configured to be implanted sub-sclerally between the coil and the array, or a retinal tack (160), and which is configured to intrude through the choroid (300) and the vitreous humour to the array (150) but so as not to pass through any part of the retina (400); and wherein the receiving coil (110) receives power and data signals by inductive coupling with a transmitting coil (120) that is located externally to the body.

11. A retinal prosthesis according to any preceding claim, wherein one or more additional, intermediate coils (130 and 132) are used to relay power or data signals, or both, from a remote transmitting coil (120) to the receiving coil (110).

12. A retinal prosthesis according to claim 11 wherein an intermediate coil (130 and 132) is configured to be located on the outer surface of the sclera (30).

13. A retinal prosthesis according to claim 11, wherein a first intermediate coil (130) is configured to be implanted in the zygomatic bone (50) for receiving power from an external primary coil (120), a second intermediate coil (132) is configured to be implanted in the orbit of the ocular region for inductively relaying power from the first intermediate coil (130) to the secondary (110), and the first and second intermediate coils (130 and 132) are in wired connection.

14. A retinal prosthesis according to any preceding claim, wherein power or data signals, or both, received by the receiving coil (110) from a remote transmitting coil are automatically provided to the electrode array.

## Patentansprüche

1. Retinaprothese, die umfasst: eine Retina-Elektrodenanordnung (150), die konfiguriert ist, um in das Auge implantiert zu werden, um die Retina (400) zu stimulieren; eine Empfangsspule (110); eine elektrische Verbindung (170) zwischen der Empfangsspule und der Retina-Elektrode; wobei die Empfangsspule biegsam ist und konfiguriert ist, sich an die sklerale Krümmung anzupassen, wenn sie implantiert ist;
**dadurch gekennzeichnet, dass** die Empfangsspule (110) konfiguriert ist, um subskleral implantiert zu werden, um Leistung und/oder Datensignale induktiv zu empfangen.

2. Retinaprothese nach Anspruch 1, wobei die Retina-Elektrodenanordnung (150) konfiguriert ist, epi-retinal in der Nähe der Fovea im Makularbereich der Retina (400) angeordnet zu werden.

3. Retinaprothese nach Anspruch 1 oder 2, die ferner eine oder mehrere selbstverriegelnde Retinaklemmen (160) umfasst, die konfiguriert sind, die mechanische Befestigung der Elektrodenanordnung (150) an der epi-retinalen Position zu erleichtern, und außerdem konfiguriert sind, eine elektrische Verbindung zwischen der Sekundärspule (110) und der Elektrodenanordnung zu vervollständigen.

4. Retinaprothese nach Anspruch 3, wobei jede der Retinaklemmen (160) aus einem Isoliermaterial wie etwa einer Keramik gebildet ist und einen Leiter aufnimmt, der elektrische Kontakte an jedem Ende der Klemme überbrückt.

5. Retinaprothese nach Anspruch 3 oder 4, wobei jede der Retinaklemmen (160) dafür ausgelegt ist, Leistung und/oder Datensignale von der Sekundärspule (110) durch die Choroidea (300) und/oder die Retina zu der Elektrodenanordnung (150) zu leiten.

6. Retinaprothese nach Anspruch 4 oder 5, wobei der Leiter jeder der Retinaklemmen (160) konfiguriert ist, Datensignale in beiden Richtungen zu und von der Retinaanordnung zu führen.

7. Retinaprothese nach Anspruch 1, wobei die Retina-Elektrodenanordnung (150) konfiguriert ist, in dem suprachoroidalen Raum angeordnet zu werden.

8. Retinaprothese nach einem vorhergehenden Anspruch, wobei die Empfangsspule (110) intrinsisch biegsam ist.

9. Retinaprothese nach einem der Ansprüche 1 bis 7, wobei die Empfangsspule (110) auf einem biegsamen Substrat gebildet oder montiert ist.

10. Retinaprothese nach Anspruch 1, wobei die Empfangsspule (110) konfiguriert ist, damit sie zwischen der Sklera (30) und der Choroidea (300), beispielsweise suprachoroidal oder epi-skleral, angeordnet ist, wobei die Empfangsspule (110) mit der Elektrodenanordnung (150) unter Verwendung einer biegsamen Verdrahtung verbunden ist, die konfiguriert ist, subskleral zwischen der Spule und der Anordnung implantiert zu werden oder unter Verwendung einer Retinaklemme (160) verbunden zu werden, und die konfiguriert ist, durch die Choroidea (300) und den Glaskörper zu der Anordnung (150) einzudringen, jedoch so, dass sie nicht durch irgendeinen Teil der Retina (400) verläuft; und wobei die Empfangsspule (110) Leistung und Datensignale durch induktive Kopplung mit einer Sendespule (120), die sich außerhalb des Körpers befindet, empfängt.

11. Retinaprothese nach einem vorhergehenden Anspruch, wobei eine oder mehrere zusätzliche Zwischenspulen (130 und 132) verwendet werden, um Leistung und/oder Datensignale von einer entfernten Sendespule (120) zu der Empfangsspule (110) weiterzuleiten.

12. Retinaprothese nach Anspruch 11, wobei eine Zwischenspule (130 und 132) konfiguriert ist, um an der äußeren Oberfläche der Sklera (30) angeordnet zu werden.

13. Retinaprothese nach Anspruch 11, wobei eine erste Zwischenspule (130) konfiguriert ist, in das Jochbein (50) implantiert zu werden, um Leistung von einer externen Primärspule (120) zu empfangen, wobei eine zweite Zwischenspule (132) konfiguriert ist, in die Höhle des Okularbereichs implantiert zu werden, um Leistung von der ersten Zwischenspule (130) zu der Sekundärspule (110) induktiv weiterzuleiten, wobei die erste und die zweite Zwischenspule (130 und 132) verdrahtet verbunden sind.

14. Retinaprothese nach einem vorhergehenden Anspruch, wobei Leistung und/oder Datensignale, die durch die Empfangsspule (110) von einer entfernten Sendespule empfangen werden, automatisch für die Elektrodenanordnung bereitgestellt werden.

## Revendications

1. Une prothèse rétinienne, comprenant : un porte-électrode rétinien (150) configuré pour être implanté dans l'oeil afin de stimuler la rétine (400), une bobine de réception (110), une connexion électrique (170) entre la bobine de réception et l'électrode rétinienne, dans laquelle la bobine de réception est flexible et configurée pour se conformer à la courbure sclérale, lorsqu'elle est implantée ;
**caractérisée en ce que** la bobine de réception (110) est configurée pour être implantée de façon sous-sclérale afin de recevoir du courant ou des signaux de données, ou les deux, de manière inductive.

2. Une prothèse rétinienne selon la revendication 1, dans laquelle le porte-électrode rétinien (150) est configuré pour être situé de façon épi-rétinienne près de la fovéa dans la région maculaire de la rétine (400).

3. Une prothèse rétinienne selon la revendication 1 ou la revendication 2, comprenant en outre une ou plusieurs pointes (160) d'auto-verrouillage rétinien qui sont configurées pour faciliter le maintien mécanique du porte-électrode (150) dans la position épi-rétinienne, et qui sont également configurées pour réaliser une connexion électrique entre la bobine secondaire (110) et le porte-électrode.

4. Une prothèse rétinienne selon la revendication 3, dans laquelle chacune desdites pointes rétiniennes (160) est formée d'un matériau isolant, tel qu'une céramique, et loge un conducteur interne qui s'étend entre des contacts électriques situés à chaque extrémité de la pointe.

5. Une prothèse rétinienne selon la revendication 3 ou la revendication 4, dans laquelle chacune desdites pointes rétiniennes (160) est agencée pour conduire du courant ou des signaux de données, ou les deux, depuis la bobine secondaire (110) à travers la choroïde (300) ou la rétine (400), ou les deux, jusqu'au porte-électrode (150).

6. Une prothèse rétinienne selon la revendication 4 ou la revendication 5, dans laquelle le conducteur de chacune desdites pointes rétiniennes (160) est configuré pour transporter des signaux de données dans les deux sens, vers et depuis la matrice de la rétine.

7. Une prothèse rétinienne selon la revendication 1, dans laquelle le porte-électrode rétinien (150) est configuré pour être situé dans l'espace supra-choroïdien.

8. Une prothèse rétinienne selon l'une quelconque des revendications précédentes, dans laquelle la bobine de réception (110) est intrinsèquement flexible.

9. Une prothèse rétinienne selon l'une quelconque des revendications 1 à 7, dans laquelle la bobine de réception (110) est formée ou montée sur un substrat flexible.

10. Une prothèse rétinienne selon la revendication 1, dans laquelle la bobine de réception (110) est configurée pour être située entre la sclère (30) et la choroïde (300), par exemple de façon supra-choroïdale et épi-sclérale, la bobine de réception (110) étant reliée au porte-électrode (150) par l'utilisation d'un câblage flexible, qui est configuré pour être implanté de façon sous-sclérale entre la bobine et le porte-électrode, ou une pointe rétinienne (160), et qui est configurée pour s'introduire à travers la choroïde (300) et le corps vitré jusqu'au porte-électrode (150) mais de telle sorte qu'elle ne passe pas au travers d'aucune partie de la rétine (400), la bobine de réception (110) recevant du courant et des signaux de données par couplage inductif avec une bobine de transmission (120) qui est située à l'extérieur du corps.

11. Une prothèse rétinienne selon l'une quelconque des revendications précédentes, dans laquelle une ou plusieurs bobines intermédiaires supplémentaires (130 et 132) sont utilisées pour transmettre du courant ou des signaux de données, ou les deux, depuis une bobine de transmission (120) située à distance, vers la bobine de réception (110).

12. Une prothèse rétinienne selon la revendication 11, dans laquelle une bobine intermédiaire (130 et 132) est configurée pour être située sur la surface externe de la sclère (30).

13. Une prothèse rétinienne selon la revendication 11, dans laquelle une première bobine intermédiaire (130) est configuré pour être implanté dans l'os zygomatique (50) pour recevoir du courant depuis une bobine primaire externe (120), une deuxième bobine intermédiaire (132) étant configurée pour être implantée dans l'orbite la région oculaire pour relayer du courant de manière inductive depuis la première bobine intermédiaire (130) à la deuxième bobine (110), et les première et deuxième bobine intermédiaires (130 et 132) sont en connexion câblée.

14. Une prothèse rétinienne selon l'une quelconque des revendications précédentes, dans laquelle du courant ou des signaux de données, ou les deux, reçus par la bobine de réception (110) depuis une bobine de transmission distante sont automatiquement fournis porte-électrode.
